(19) **Europäisches Patentamt**

**European Patent Office**

**Office européen des brevets**

(11) **EP 1 167 343 B1**

(12) **EUROPÄISCHE PATENTSCHRIFT**

(45) Veröffentlichungstag und Bekanntmachung des Hinweises auf die Patenterteilung:
**21.05.2003 Patentblatt 2003/21**

(51) Int Cl.$^7$: **C07C 209/78**

(21) Anmeldenummer: **01114057.1**

(22) Anmeldetag: **08.06.2001**

(54) **Verfahren zur Herstellung eines aromatischen Polyamingemisches**

Process for the production of an aromatic polyamine mixture

Procédé pour la production d'un mélange de polyamines aromatiques

(84) Benannte Vertragsstaaten:
**AT BE CH CY DE DK ES FI FR GB GR IE IT LI LU MC NL PT SE TR**

(30) Priorität: **28.06.2000 DE 10031540**

(43) Veröffentlichungstag der Anmeldung:
**02.01.2002 Patentblatt 2002/01**

(73) Patentinhaber: **BASF AKTIENGESELLSCHAFT**
**67056 Ludwigshafen (DE)**

(72) Erfinder:
• **Müller, Christian**
  **68167 Mannheim (DE)**
• **Sohn, Martin, Dr.**
  **68229 Mannheim (DE)**
• **Bettendorf, Carl**
  **2950 Kapellen (BE)**
• **van den Abeel, Peter**
  **2930 Brasschaat (BE)**
• **Penzel, Ulrich, Dr.**
  **01945 Tettau (DE)**
• **Kraus, Rupert, Dr.**
  **67165 Waldsee (DE)**
• **Thiele, Kai, Dr.**
  **01987 Schwarzheide (DE)**
• **Ströfer, Eckhard, Dr.**
  **68163 Mannheim (DE)**

(56) Entgegenhaltungen:
EP-A- 0 283 757       DE-A- 19 804 915
US-A- 3 860 637       US-A- 4 924 028
US-A- 5 359 141

**Beschreibung**

Die Erfindung betrifft ein Verfahren zur Herstellung eines aromatischen Polyamingemisches, das im wesentlichen 4,4'-Methylen(diphenylamin) und höhere Homologe von Methylen-(diphenylamin) enthält.

Die Herstellung von Methylen(diphenylamin) (im folgenden als MDA bezeichnet) erfolgt üblicherweise durch kontinuierliche oder diskontinuierliche Umsetzung von Anilin mit Formaldehyd in Gegenwart von sauren Katalysatoren. Bei dieser Umsetzung entsteht ein Gemisch aus überwiegend 4,4'-MDA, 2,4'-MDA, 2,2'-MDA und höheren Homologen von MDA, das als "Roh-MDA" bezeichnet wird. Die Isomeren 2,4'-MDA und 2,2'-MDA sind jedoch oft unerwünscht, da bei einer Folgeumsetzung mit Phosgen die Verbindungen 2,4'-Methylen-(diphenylisocyanat) (2,4'-MDI) und 2,2'-MDI gebildet werden. Diese Verbindungen weisen ortho-ständige Isocyanatgruppen auf, die bei einer Urethanisierungsreaktion mit Polyolen nur unvollständig abreagieren.

Zur Verringerung des Gehalts an 2,4'-MDA und 2,2'-MDA im Roh-MDA sind verschiedene Verfahren bekannt.

EP-A-0283757 beschreibt ein Verfahren zur Herstellung eines Polyamingemisches, bei dem als Zwischenprodukt gebildete Aminobenzylamine (ABA) vor ihrer Umlagerung zu dem gewünschten Polyamingemisch ein anilinfreies Gemisch aus verschiedenen MDA-Isomeren und oligomeren Polymethylenpolyphenylpolyaminen (PMDA) zugegeben wird. Nachteilig bei diesem Verfahren ist die nicht ausreichende Abnahme an 2,4'-MDA und 2,2'-MDA und die insgesamt niedrige MDA Selektivität im erhaltenen Roh-MDA.

DE-A-19513068 offenbart ein Verfahren zur Fraktionierung und Reinigung von aromatischen Polyamingemischen. Nachteilig bei diesem Verfahren ist, dass man unterschiedliche Aminfraktionen erhält, die im Falle einer MDI-Herstellung getrennt voneinander phosgeniert werden müssen.

US 3,860,637 beschreibt ein Verfahren zur Herstellung von MDA, wobei ein Strom von 2,2'-MDA und 2,4'-MDA vom Reaktionsprodukt abgetrennt und in den Amin-Formaldehyd-Strom rückgeführt werden. Das beschriebene Verfahren hat jedoch nur eine mäßige Selektivität von 4,4'-MDA bezogen auf 2-Kern-MDA.

Aufgabe der vorliegenden Erfindung war es daher, ein verbessertes Verfahren zur Herstellung eines aromatischen Polyamingemisches durch Umsetzung von Anilin mit Formaldehyd bereitzustellen, welches im wesentlichen 4,4'-Methylen(diphenylamin) und höhere Homologe von Methylen(diphenylamin) enthält und das eine möglichst hohe Selektivität von 4,4'-MDA bezogen auf 2-Kern-MDA aufweist, wobei unter 2-Kern-MDA 2,2'-MDA, 2,4'-MDA und 4,4'-MDA zu verstehen ist, d.h.

$$\text{Selektivität (4,4'-MDA)} = n\,(4,4'\text{-MDA}) / [n\,(2,2'\text{-MDA}) + n\,(2,4'\text{-MDA}) + n\,(4,4'\text{-MDA})],$$

wobei n die jeweilige molare Menge darstellt.

Die Aufgabe konnte dadurch gelöst werden, daß von einem Rohproduktgemisch, das aus der Umsetzung von Anilin mit Formaldehyd resultiert, ein Gemisch aus 2,4'- und 2,2'-Methylen(diphenylamin) abgetrennt, rückgeführt und mit einem Teilstrom des Formaldehyds vermischt wird. Es hat sich unerwarteter Weise gezeigt, dass die Rückführung des Gemisches aus 2,4'-MDA und 2,2'-MDA die Neubildung von 4,4'-MDA aus Anilin und Formaldehyd nur geringfügig beeinflußt und dass dadurch die Selektivität von 4,4'-MDA bezogen auf 2-Kern-MDA gesteigert werden konnte.

Gegenstand der Erfindung ist daher Verfahren zur Herstellung eines aromatischen Polyamingemisches, das 4,4'-Methylen(diphenylamin) und höhere Homologe von Methylen(diphenylamin) enthält, durch
Umsetzung von Anilin mit Formaldehyd zu einem Rohproduktgemisch (Stufe I), wobei Stufe I in einer Anlage durchgeführt wird, die eine Mischzone (a), eine Kondensationszone (b), eine Umlagerungszone (c) und gegebenenfalls eine Nachreaktionszone (d) umfaßt, und
Abtrennung eines Gemisches aus 2,2'-Methylendi(phenylamin) und 2,4'-Methylendi(phenylamin) von dem Rohproduktgemisch (Stufe II), dadurch gekennzeichnet, dass man den eingesetzten Formaldehyd in mindestens zwei Teile aufteilt, wobei ein Teil der Kondensationszone (b) zugeführt wird und ein Teil mit dem aus Stufe II abgetrennten Gemisch aus 2,2'-Methylendi(phenylamin) und 2,4'-Methylendiphenylamin vermischt und der Umlagerungszone c) zugeführt wird.

Das Polyamingemisch der vorliegenden Erfindung enthält im wesentlichen 4,4'-Methylen(diphenylamin) und höheren Homologen von Methylen(diphenylamin). Darunter ist im Rahmen dieser Erfindung zu verstehen, dass das Polyamingemisch zumeist einen Gehalt an 4,4'-MDA und höheren Homologen von MDA von größer 90 Gew.-%, bevorzugt größer 95 Gew.-%, besonders bevorzugt von größer 99 Gew.-% aufweist. Der Gehalt an 2,2'-MDA und 2,4'-MDA ist zumeist kleiner als 10 Gew.-%, bevorzugt kleiner als 5 Gew.-% und besonders bevorzugt kleiner als 1 Gew.-%.

Das Gemisch aus 2,2'-Methylendi(phenylamin) und 2,4'-Methylendi-(phenylamin), das aus dem Rohproduktgemisch abgetrennt und in Stufe I rückgeführt wird, enthält im allgemeinen mindestens 55 Gew.-% 2,2'-MDA und 2,4'-MDA, bevorzugt mindestens 75 Gew.-% 2,2'-MDA und 2,4'-MDA, stärker bevorzugt mindestens 90 Gew.-% und besonders bevorzugt mindestens 95 % 2,2'-MDA und 2,4'-MDA. Neben 2,2'-MDA und 2,4'-MDA kann das Gemisch, das in Stufe I rückgeführt wird, beispielsweise auch Anilin, 4,4-MDA und Polymer-MDA enthalten.

Im erfindungsgemäßen Verfahren wird ein Gemisch aus 2,2'-MDA und 2,4'-MDA aus Stufe II in Stufe I rückgeführt. Darunter ist auch zu verstehen, dass das abgetrennte Gemisch aus 2,2'-MDA und 2,4'-MDA nicht direkt zurückgeführt wird, sondern in dafür geeigneten Vorrichtungen zwischengelagert werden kann. Ferner ist es beispielsweise auch möglich, das abgetrennte Gemisch aus 2,2'-MDA und 2,4'-MDA in eine Stufe I einer weiteren Anlage wieder zuzuführen oder das aus einer weiteren Anlage abgetrennte Gemisch aus 2,2'-MDA und 2,4'-MDA in Stufe I zuzuführen.

In der vorliegenden Erfindung erfolgt die Umsetzung von Anilin mit Formaldehyd zu einem Rohproduktgemisch (Stufe (I)) in einer Anlage mit folgenden Reaktionszonen:

(a) Vermischen von Anilin oder gegebenenfalls eines Gemisches aus Anilin und Katalysator mit Formaldehyd in einer geeigneten Mischvorrichtung. Diese Reaktionszone wird als Mischzone bezeichnet.

(b) Reaktion in einer Reaktionsvorrichtung zumeist in einem Temperaturbereich von 20 bis 100°C, bevorzugt von 30 bis 60°C. Hierbei erfolgt überwiegend eine Kondensation der Edukte zu sogenannten Aminobenzylaminen (ABAs), die als Zwischenprodukt auftreten. Diese Reaktionszone der Stufe (I) wird im Rahmen dieser Erfindung als Kondensationszone bezeichnet.

(c) Reaktion in einer Reaktionsvorrichtung zumeist in einem Temperaturbereich von 60 bis 140°C, bevorzugt von 70 bis 100°C. Hierbei erfolgt überwiegend eine Umlagerung der als Zwischenprodukt gebildeten Aminobenzylamine zu Roh-MDA. Diese Zone wird im Rahmen dieser Erfindung als Umlagerungszone bezeichnet.

(d) Reaktion in einer Reaktionsvorrichtung zumeist in einem Temperaturbereich von 70 bis 180°C, bevorzugt von 80 bis 120°C. Hierbei erfolgt überwiegend eine Vervollständigung der vorstehend erwähnten Umlagerungsreaktion zu Roh-MDA. Diese Reaktionszone wird im Rahmen dieser Erfindung als Nachreaktionszone bezeichnet. Stufe d) ist optional.

Als Mischvorrichtungen für die Reaktionszone (a) sind beispielsweise Mischpumpen, Düsen und statische Mischer geeignet.

Als Reaktionsvorrichtung für die Reaktionszonen (b) bis (d) sind beispielsweise Rohrreaktoren, Rührreaktoren und Reaktionskolonnen geeignet. Ferner können die Reaktionszonen (b) bis (d) der Stufe (I) in einem einzigen Reaktor, der gegebenenfalls unterschiedliche Temperaturbereiche aufweist, durchgeführt werden. Dies ist beispielsweise in einer Bodenkolonne möglich.

Das Rohproduktgemisch aus der Reaktionszone (d) wird zur Abtrennung von Nebenkomponenten in eine Aufarbeitungszone (e) überführt. Nebenkomponenten sind überwiegend nicht umgesetztes Anilin, Wasser, und gegebenenfalls Lösungsmittel. Wird ein saurer Katalysator eingesetzt, so kann ferner in der Aufarbeitungszone (e) eine Neutralisation des Katalysators, bevorzugt mit wässriger NaOH, und eine anschließende Abtrennung der entstehenden Salze in der Wasserphase erfolgen. Ebenfalls erfolgt in der Aufarbeitungszone (e) die Abtrennung des Gemisches aus den Verbindungen 2,2'-MDA und 2,4'-MDA (Stufe (II)). Diese erfolgt bevorzugt, nachdem die Nebenkomponenten bereits abgetrennt wurden.

Als geeignete Vorrichtung zur Abtrennung der Nebenkomponenten haben sich Rührreaktoren und Extraktionskolonnen erwiesen.

Die Abtrennung des Gemisches aus 2,2'-MDA und 2,4'-MDA kann durch verschiedene Verfahren, wie beispielsweise Kristallisation oder Destillation, erfolgen. Besonders vorteilhaft kann ein Destillationsverfahren eingesetzt werden.

Zum destillativen Abtrennen von 2,2'-MDA und 2,4'-MDA kann eine Destillationskolonne eingesetzt werden. Geeignete Destillationskolonnen weisen im allgemeinen mindestens 40, bevorzugt mindestens 50 theoretische Trennstufen, einen Temperaturverlauf von 180 bis 280°C, einen Kopfdruck von 0,1 bis 10 mbar und einen Sumpfdruck von 8 bis 20 mbar auf. Ferner hat es sich als besonders vorteilhaft erwiesen, zur Abtrennung eine Destillationskolonne, umfassend eine druckverlustarme Gewebepackung gemäß DE-A-19605286, zu verwenden.

Gemäß dem erfindungsgemäßen Verfahren wird das in Stufe II abgetrennte Gemisch aus 2,2'-Methylendi(phenylamin) und 2,4'-Methylendi(phenylamin) rückgeführt und in Stufe I wieder dem Verfahren zugesetzt.

Es kann vorteilhaft sein, bei dem erfindungsgemäßen Verfahren mindestens einen Katalysator einzusetzen. Bevorzugt wird ein saurer Katalysator verwendet. Handelt es sich dabei um einen homogenen Katalysator, so wird dieser bevorzugt im Gemisch mit Anilin zugegeben. Als homogene Katalysatoren sind Mineralsäuren, besonders Salzsäure, Schwefelsäure und Phosphorsäure geeignet. Wird als homogener Katalysator Chlorwasserstoff verwendet; so kann dieser gasförmig oder als wässrige Lösung eingesetzt werden.

Handelt es sich um einen Heterogenkatalysator, so wird dieser zumeist in die Reaktionsvorrichtungen der Zonen (b) bis (d) der Stufe (I) eingebracht.

Als heterogene Katalysatoren sind Ionenaustauscher, Tone, Zeolithe, Silica-Alumina-, Wolfram- oder Molybdänoxide auf diversen Trägern wie beispielsweise $TiO_2$, $ZrO_2$, $HfO_2$, $SnO_2$ und $Al_2O_3$ vorteilhaft und saure Ionenaustauscher besonders vorteilhaft. Die genannten Katalysatoren können gegebenenfalls in beliebiger Kombination eingesetzt werden.

Alternativ ist es auch möglich, Anilin in Form eines sauren Salzes in das erfindungsgemäße Verfahren einzubringen. Bevorzugt liegt dabei das Amin (a) in Form eines Hydrochlorids, Sulfats oder Phosphats vor.

Weiterhin kann $CO_2$ unter überkritischen Bedingungen als Katalysator dienen.

Wird ein homogener Katalysator verwendet, so liegt zumeist ein molares Verhältnis Anilin : Katalysator von 1 : 0,6 bis 1 : 0,001, bevorzugt von 1 : 0,3 bis 1: 0,05 vor. Im Fall der heterogenen Katalyse liegt der Katalysator bezogen auf Anilin mit 1 bis 99 Gew.-%, bevorzugt 20 bis 60 Gew.-% vor. Es ist auch möglich, ein Gemisch aus homogenen und heterogenen Katalysator zu verwenden.

Das erfindungsgemäße Verfahren kann ferner in Gegenwart eines Lösungsmittels durchgeführt werden. Besonders geeignet sind Ether, gegebenenfalls mit Halogenatomen substituierte aromatische und aliphatische Lösungsmittel, Wasser und Gemische davon. Beispiele hierfür sind Benzol, Toluol, Monochlorbenzol, Dichlorbenzol, Dimethylformamid (DMF), Tetrahydrofuran (THF) und Diethylisophthalat (DEIP). Besonders bevorzugt wird Monochlorbenzol verwendet. Ferner ist es möglich, Anilin selbst als Lösungsmittel einzusetzen.

Formaldehyd kann dem erfindungsgemäßen Verfahren in Form von monomeren Formaldehyd und/oder in Form von höheren Homologen, sogenannte Poly(oxymethylen)glykole, zugeführt werden.

Das molare Verhältnis Anilin : Formaldehyd beträgt im allgemeinen 1,75 bis 10 : 1, bevorzugt 2 bis 4 : 1.

Im erfindungsgemäßen Verfahren wird von der in Stufe (I) gebildeten Menge des Gemisches aus 2,2'-MDA und 2,4'-MDA mindestens 50 Gew.-%, bevorzugt > 80 Gew.-%, mehr bevorzugt > 95 Gew.-% in Stufe II abgetrennt und in Stufe I rückgeführt.

Das erfindungsgemäße Verfahren wird bevorzugt kontinuierlich durchgeführt.

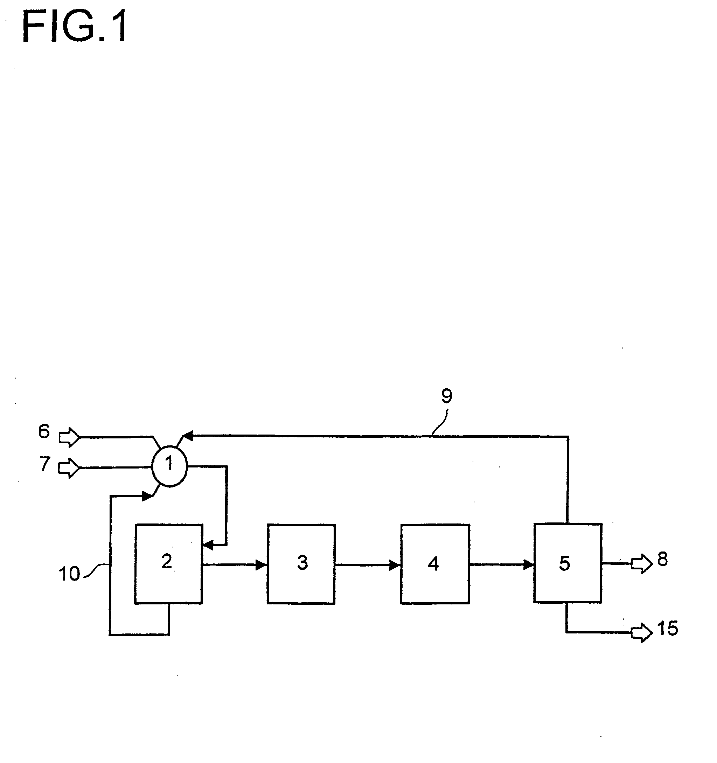

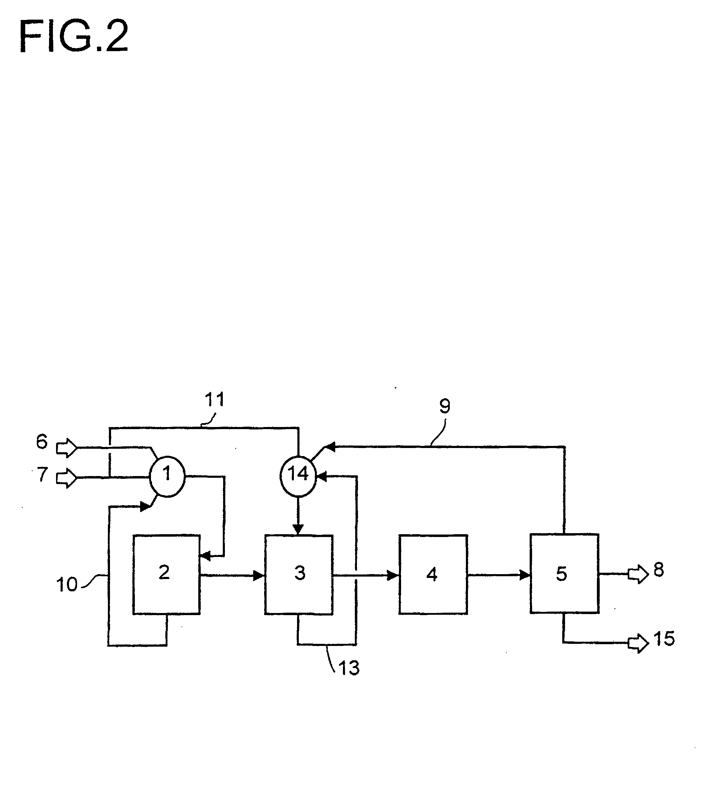

Die Figuren 1 und 2 veranschaulichen eine Ausführungsform gemäß US 3,860,637 und eine bevorzugte Ausführungsform des erfindungsgemäßen Verfahrens.(Figur 2)

In den Figuren bedeuten:

1    Mischzone (a)
2    Kondensationszone (b)
3    Umlagerungszone (c)
4    Nachreaktionszone (d)
5    Aufarbeitungszone (e)
6    Zufuhr von Anilin, gegebenenfalls im Gemisch mit Katalysator
7    Zufuhr von Formaldehyd
8    Austrag von 4,4'-MDA und höheren Homologen von MDA
9    Rückführung des Gemisches aus 2,2'-MDA und 2,4'-MDA
10   Teilrückführung des Gemisches der Kondensationszone
11   Zufuhr Formaldehydsplit
13   Teilrückführung des Gemisches der Umlagerungszone
14   Mischvorrichtung
15   Austrag Nebenkomponenten
16   Reaktionszone
17   Teilrückführung des Gemisches der Reaktionszone

Figur 1 veranschaulicht eine Ausführungsform gemäß US 3,860,637. Hierbei wird das abgetrennte Gemisch aus 2,2'-MDA und 2,4'-MDA rückgeführt (9) und mit Anilin, dem ein saurer Katalysator beigemischt ist, und Formaldehyd in einer Mischzone (a) vermischt. Bevorzugt wird hier eine Mischkammer einer Reaktionsmischpumpe oder eine Düse, die der Kondensationszone (b) vorgeschaltet sind, verwendet, so dass möglichst rasch eine vollständige Durchmischung eintritt. Eine rasche Durchmischung vermindert dabei unerwünschte Parallelreaktionen.

Figur 2 veranschaulicht eine bevorzugte erfindungsgemäße Ausführungsform. Hier wird das abgetrennte Gemisch aus 2,2'-MDA und 2,4'-MDA rückgeführt (9), mit einem Teil des Formaldehyds vermischt und der Umlagerungszone (c) zugeführt. Der restliche Formaldehydanteil wird mit dem Anilin, dem ein saurer Katalysator beigemischt ist, in der Mischzone (a) vermischt und der Kondensationszone (b) zugeführt (Formaldehydsplit). Als Mischvorrichtung sind eine Mischkammer einer Reaktionsmischpumpe oder eine Düse geeignet. Das Verhältnis Formaldehydzugabe Kondensationszone (b) : Umlagerungszone (c) beträgt im allgemeinen 0,01 bis 99 : 1, bevorzugt 0,1 bis 10: 1, mehr bevorzugt 0,3 bis 3:1.

Hierbei stellt beispielsweise ein Rohr- oder Rührreaktor eine Reaktionszone 16 dar, die eine Kondensations-, Umlagerungs- und Nachreaktionszone umfaßt. Üblicherweise wird Anilin oder Anilinhydrochlorid vorgelegt und eine Formalinlösung zudosiert. Die Zugabe kann direkt in den Kessel oder über eine geeignete Mischeinheit erfolgen. Im allgemeinen wird anschließend ein geeignetes Temperaturprofil angelegt. Nach erfolgter Kondensations- und Umlagerungsreaktion erfolgt Entleeren des Reaktors und eine anschließende Aufarbeitung und Abtrennung des Gemisches aus 2,2'-MDA und 2,4'-MDA.

Die vorliegende Erfindung soll anhand der nachfolgenden Beispiele veranschaulicht werden.

Beispiele

Vergleichsbeispiel 1: Rückführung von 2,4'-/2,2'-MDA in den Kondensationsteil einer kontinuierlichen MDA Anlage

In einer kontinuierlich betriebenen Miniplant-Anlage gemäß Figur 1 wurden in einer Mischpumpe (1) folgende Stoffströme vermischt:

$$1439 \text{ g/h Anilin} + 304 \text{ g/h wässrige Salzsäure (30\%ig)} \tag{6}$$

$$313 \text{ g/h wässrige Formalinlösung (49\%ig)} \tag{7}$$

Zusätzlicher Stoffstrom des in Stufe II abgetrennten und rückgeführten Gemisches:

$$77 \text{ g/h MDA} \tag{9}$$

Zusammensetzung des Rückführstromes:

| | |
|---|---|
| 0,3 % | 2,2'-MDA |
| 99,4 % | 2,4'-MDA |
| 0,3 % | 4,4'-MDA |

Die Mischeinheit (1) befindet sich im Umpumpkreislauf (10) des ersten von zwei in Reihe geschalteten Rührkesselreaktoren (2 und 3). Die Reaktionsmischung gelangt nach verlassen des ersten Reaktors (2) in den zweiten Rührkesselreaktor (3) und anschließend in drei hintereinander geschaltete Rohrreaktoren (4) mit ansteigendem Temperaturprofil.

Die mittleren Verweilzeiten der einzelnen Reaktoren und die dazugehörigen Reaktortemperaturen sind der folgenden Tabelle zu entnehmen:

| Reaktor | Verweilzeit [min] | Temperatur [°C] |
|---|---|---|
| 1. Rührreaktor | 30 | 50 |
| 2. Rührreaktor | 30 | 70 |
| 1. Rohrreaktor | 30 | 100 |
| 2. Rohrreaktor | 30 | 120 |
| 3. Rohrreaktor | 30 | 120 |

Aufarbeitung

Die ausreagierte Reaktionsmischung wird nach Verlassen des letzten Rohrreaktors mit wässriger Natronlauge (25%ig) neutralisiert. Nach Abtrennung der wässrigen Phase wird die organische Phase mit Wasser gewaschen und anschließend destilliert.

In der folgenden Tabelle sind die Massenströme der erhaltenen Produkte zwischen Nachreaktionszone (4) und Aufarbeitungszone (5) der beiden betrachteten Experimente gegeben:

| Produkt | Vgl.-Beispiel 1 [g/h] |
|---|---|
| 2,2'- und 2,4'-MDA | 125,3 |
| 4,4'-MDA | 601,0 |
| 3 Kern MDA | 226,5 |
| 4 Kern MDA | 69,0 |
| 5 Kern MDA | 12,2 |
| Summe | 1034,0 |

In der folgenden Tabelle sind die Massenströme der erhaltenen Produkte nach der Aufarbeitungszone (5) der beiden betrachteten Experimente gegeben, wenn gerade 77 g/h (0,3 % 2,2'-MDA, 99,4 % 2,4'-MDA, 0,3 % 4,4'-MDA) zurückgeführt werden:

| Produkt Produkt | Vgl.-Beispiel 1 mit Aufarbeitung (Destillation) [g/h] |
|---|---|
| 2,2'- und 2,4'-MDA | 48,5 |
| 4,4'-MDA | 600,8 |
| 3 Kern MDA | 226,5 |
| 4 Kern MDA | 69,0 |
| 5 Kern MDA | 12,2 |

Selektivität von 4,4'-MDA bezogen auf 2-Kern MDA:
Vgl.-Beispiel 1:

$$S_{2\text{-}Kern}^{4,4'\text{-}} = 92,5\ \%$$

Beispiel 2: Rückführung von 2,4'-/2,2'-MDA in den Kondensationsteil einer kontinuierlichen MDA-Anlage mit Formaldehydsplit

In einer kontinuierlich betriebenen Miniplant-Anlage gemäß Figur 2 wurden in einer Mischpumpe (1) folgende Stoff ströme vermischt:

1419 g/h    Anilin + 272 g/h wässrige Salzsäure (30%ig) (6)
350 g/h    wässrige Formalinlösung (49%ig), (75 % in den ersten Reaktor (2) und 25 % in den zweiten Reaktor (3))

Zusätzlicher Stoffstrom des in Stufe II abgetrennten und rückgeführten Gemisches:

110 g/h MDA                                                                                (9)

Zusammensetzung des Rückführstromes:

| | |
|---|---|
| 0,1 % | 2,2'-MDA |
| 99,5 % | 2,4'-MDA |
| 0,4 % | 4,4'-MDA |

Die Mischeinheit (1) befindet sich im Umpumpkreislauf (10) des ersten von zwei in Reihe geschalteten Rührkesselreaktoren (2 und 3). Die Reaktionsmischung gelangt nach verlassen des ersten Reaktors (2) in den zweiten Rührkesselreaktor (3) und anschließend in drei hintereinander geschaltete Rohrreaktoren (4) mit ansteigendem Temperaturprofil.

Die mittleren Verweilzeiten der einzelnen Reaktoren und die dazugehörigen Reaktortemperaturen sind der folgenden Tabelle zu entnehmen:

| Reaktor | Verweilzeit [min] | Temperatur [°C] |
|---|---|---|
| 1. Rührreaktor | 30 | 60 |
| 2. Rührreaktor | 30 | 90 |
| 1. Rohrreaktor | 30 | 100 |
| 2. Rohrreaktor | 30 | 120 |
| 3. Rohrreaktor | 30 | 120 |

Die Aufarbeitung erfolgt wie in Beispiel 1 beschrieben.

In der folgenden Tabelle sind die Massenströme der erhaltenen Produkte zwischen Nachreaktionszone (4) und Aufarbeitungszone (5) der beiden betrachteten Experimente gegeben:

| Produkt | Beispiel 2 [g/h] |
|---|---|
| 2,2'- und 2,4'-MDA | 144,6 144,6 |
| 4,4'-MDA | 559,5 |
| 3 Kern MDA | 267,7 |
| 4 Kern MDA | 92,1 |
| 5 Kern MDA | 13,4 |
| Summe | 1077,3 |

In der folgenden Tabelle sind die Massenströme der erhaltenen Produkte nach der Aufarbeitungszone (5) der beiden betrachteten Experimente gegeben, wenn gerade 110 g/h (0,1 % 2,2'-MDA, 99,5 % 2,4'-MDA, 0,4 % 4,4'-MDA) zurückgeführt werden:

| Produkt | Beispiel 2 mit Aufarbeitung (Destillation) [g/h] |
|---|---|
| 2,2'- und 2,4'-MDA | 35,0 |
| 4,4'-MDA | 559,1 |
| 3 Kern MDA | 267,7 |
| 4 Kern MDA | 92,1 |
| 5 Kern MDA | 13,4 |

Selektivität von 4,4'-MDA bezogen auf 2-Kern MDA:

Beispiel 2:

$$S_{2\text{-}Kern}^{4,4'\text{-}} = 94.1\%$$

Im erfindungsgemäßen Beispiel 2 konnte die Selektivität gegenüber dem Vergleichsbeispiel 1 von 92,5 auf 94,1 % gesteigert werden. Dies entspricht einer Abnahme der "unerwünschten" Bestandteile von 7,5 % auf 5,9 %, d. h. eine Verringerung um 20 %.

**Patentansprüche**

1. Verfahren zur Herstellung eines aromatischen Polyamingemisches, das 4,4'-Methylen(diphenylamin) und höhere Homologe von Methylen(diphenylamin) enthält, durch Umsetzung von Anilin mit Formaldehyd zu einem Rohproduktgemisch (Stufe I), wobei Stufe I in einer Anlage durchgeführt wird, die eine Mischzone (a), eine Kondensationszone (b), eine Umlagerungszone (c) und gegebenenfalls eine Nachreaktionszone (d) umfaßt, und Abtrennung eines Gemisches aus 2,2'-Methylendi(phenylamin) und 2,4'-Methylendi(phenylamin) von dem Rohproduktgemisch (Stufe II),
**dadurch gekennzeichnet, dass** man den eingesetzten Formaldehyd in mindestens zwei Teile aufteilt, wobei ein Teil der Kondensationszone (b) zugeführt wird und ein Teil mit dem aus Stufe II abgetrennten Gemisch aus 2,2'-Methylendi(phenylamin) und 2,4'-Methylendiphenylamin vermischt und der Umlagerungszone c) zugeführt wird.

2. Verfahren nach Anspruch 1, **dadurch gekennzeichnet, dass** die Abtrennung des Gemisches aus 2,2'-Methylendi(phenylamin) und 2,4'-Methylendi(phenylamin) durch Destillation erfolgt.

3. Verfahren nach Anspruch 2, **dadurch gekennzeichnet, dass** die Destillation mittels einer Kolonne, die eine theoretische Trennstufenzahl von mindestens 50 aufweist, erfolgt.

4. Verfahren nach einem der Ansprüche 1 bis 3, **dadurch gekennzeichnet, dass** in Stufe (I) die Umsetzung von Anilin mit Formaldehyd in Gegenwart eines Katalysators erfolgt.

**Claims**

1. A process for the preparation of an aromatic polyamine mixture which contains 4,4'-methylenedi(phenylamine) and higher homologs of methylenedi(phenylamine) by reaction of aniline with formaldehyde to give a crude product mixture (Stage I), where Stage I is carried out in a plant which comprises a mixing zone (a), a condensation zone (b), a rearrangement zone (c) and, if required, a secondary reaction zone (d), and separation of a mixture of 2,2'-methylenedi(phenylamine) and 2,4'-methylenedi(phenylamine) from the crude product mixture (Stage II),
wherein the formaldehyde used is divided into at least two parts, one part being fed to the condensation zone (b) and one part being mixed with the mixture of 2,2'-methylenedi(phenylamine) and 2,4'-methylenediphenylamine, separated off from stage II, and being fed to the rearrangement zone (c).

2. A process as claimed in claim 1, wherein the separation of the mixture of 2,2'-methylenedi(phenylamine) and 2,4'-methylenedi(phenylamine) is effected by distillation.

3. A process as claimed in claim 2, wherein the distillation is effected by means of a column which has at least 50 theoretical plates.

4. A process as claimed in any of claims 1 to 3, wherein in Stage (I) the reaction of aniline with formaldehyde is effected in the presence of a catalyst.

**Revendications**

1. Procédé de préparation d'un mélange de polyamines aromatiques qui contient de la 4,4'-mèthylène(diphénylamine) et des homologues supérieurs de la méthylène(diphénylamine), par réaction de l'aniline avec le formaldéhyde pour obtenir un mélange de produit brut (étape I), l'étape I étant réalisée dans une installation qui comprend une zone de mélange (a), une zone de condensation (b), une zone de réarrangement (c), et éventuellement une zone de post-réaction (d), et par séparation d'un mélange de 2,2'-méthylène-di(phénylamine) et de 2,4'-méthylène-di(phénylamine) d'avec le mélange de produit brut (étape II),
**caractérisé en ce que** l'on partage le formaldéhyde mis en oeuvre en au moins deux parties, une partie étant introduite dans la zone de condensation (b) et une partie étant mélangée avec le mélange séparé de 2,2'-méthylène-di(phénylamine) et de 2,4'-méthylène-di(phénylamine) issu de l'étape II et introduite dans la zone de réarrangement (c).

2. Procédé selon la revendication 1, **caractérisé en ce que**, la séparation du mélange de 2,2'-méthylène-di(phénylamine) et de 2,4'-mèthylène-di(phénylamine) se réalise par distillation.

3. Procédé selon la revendication 2, **caractérisé en ce que** la distillation se réalise au moyen d'une colonne qui présente au moins un nombre théorique de plateaux de séparation d'au moins 50.

4. Procédé selon l'une quelconque des revendications 1 à 3, **caractérisé en ce que** dans l'étape (I), la réaction de l'aniline avec le formaldéhyde se réalise en présence d'un catalyseur.

# FIG.1

# FIG.2